# EUROPÄISCHE PATENTSCHRIFT

(11) **EP 1 095 626 B1**
(45) Veröffentlichungstag und Bekanntmachung des Hinweises auf die Patenterteilung: **24.02.2016**
(21) Anmeldenummer: 00810915.9
(22) Anmeldetag: 04.10.2000
(51) Int. Cl.: A61B 17/72

(54) **RADIUSMARKNAGEL**
INTRAMEDULLARY NAIL FOR THE RADIUS
CLOU CENTROMÉDULLAIRE POUR LE RADIUS

(30) Priorität: 01.11.1999 EP 99810989
(43) Veröffentlichungstag der Anmeldung: 02.05.2001
(73) Patentinhaber: Zimmer GmbH, 8404 Winterthur (CH)
(72) Erfinder: Gnos, Robert, 8932 Mettmenstetten (CH); Guelmi Dr. Kamel, Chirurgien des Hôpitaux de Paris, 75730 Paris Cedex 15 (FR)
(74) Vertreter: Manitz, Finsterwald & Partner GbR

(56) Entgegenhaltungen:
- EP-A- 0 202 141
- EP-A1- 0 548 658
- WO-A-99/35989
- DE-U- 9 115 201
- US-A- 4 622 959

## Beschreibung

Die Erfindung handelt von einem Radiusmarknagel, den es bisher in der vorgeschlagenen Form nicht gab.

Knochenbrüche in der Nähe des Handgelenks treten relativ häufig auf. Gerade bei älteren Menschen wird das Gefühl für das Gleichgewicht schlechter, die Hände werden mehr zur Abstützung benutzt und gleichzeitig tritt eine Schwächung der Knochen ein. Die Folge sind Brüche am Radiusknochen in der Nähe der Artikulationsflächen zum Handgelenk, d.h. zum Scapahoid und zum Lunatum. Diese Brüche treten als Scherung unterhalb der Gelenkflächen auf oder gehen quer durch die Gelenkflächen. Die bisherigen Behandlungsmethoden bestanden im Anbringen von Knochenplatten und/oder Knochenschrauben oder aber im Anbringen von einem Gipsverband mit dem Risiko, dass die Knochenfragmente nicht gerade in einer optimalen Stellung zusammenwachsen.

DE 91 15 201 U1 offenbart einen intramedullären Knochennagel, der für die Versorgung von Brüchen des Radius geeignet ist. Die Krümmung des Nagels entspricht der anatomischen Krümmung des Radius. Eine in einem distalen Endbereich vorgesehene Querbohrung erstreckt sich annähernd senkrecht zur Krümmungsebene des Nagels. Am distalen Ende sind zwei diametral gegenüberliegende Aussparungen vorgesehen, die mit einem Zielgerät zur Ermittlung der Lage der Querbohrung zusammenwirken können.

Aufgabe der Erfindung ist es eine sichere Schienung für Brüche des Radiusknochens in der Nähe vom Handgelenk zu schaffen. Diese Aufgabe wird gelöst durch einen Radiusmarknagel mit einer Krümmung seiner Längsachse entsprechend einem mittleren Krümmungsradius 100 mm < R < 1 000 mm, mit einem Befestigungskopf für eine Zielvorrichtung und mit einer schräggestellten Querbohrung, die in einer Distanz von weniger als 30 mm vom Ende des Befestigungskopfes entfernt, die Längsachse kreuzt.

Der Vorteil dieses Marknagels liegt darin, dass er ausserhalb der Gelenkfläche für den Scapahoid am Processus styloideus einbringbar ist und unmittelbar unterhalb der Gelenkflächen im Bereich der grössten Querausdehnung das Einbringen einer Knochenschraube zur Fixation und Sicherung erlaubt. Dabei dient der im Radiusknochen verankerte Marknagel, als Referenz für zu befestigende Knochenbruchstücke. Ein weiterer Vorteil liegt darin, dass durch die Schiebung von innen nur kleine äussere Verletzungen für den Eintritt von Marknagel und für Sicherungsschrauben angebracht werden müssen.

Die abhängigen Patentansprüche 2 bis 10 stellen vorteilhafte Weiterbildungen der Erfindung dar. So ist es vorteilhaft, verschieden lange Radiusmarknägel mit Längen von 50 bis 200 mm und mit verschiedenen Durchmessern von 3 bis 10 mm vorzusehen, um das Spektrum von unterschiedlichen Patienten abzudecken. Um den Befestigungskopf für die Befestigung einer Einschlag- und Zielvorrichtung und für das Anbringen der schräggestellten Querbohrung nicht unnötig zu schwächen, wird er mit einem grösseren Aussendurchmeser als der Rest des Radiusmarknagels versehen. Die Querbohrung kann mit der Schaftachse des Befestigungskopfes einen Winkel α zwischen 40° und 70° bilden, um mit der Querbohrung einen Bereich nahe unterhalb der Gelenkpfannen im distalen Bereich des Radiusknochens zu kreuzen und um auch Knochenbruchstücke von Brüchen durch die Gelenkpfannen zu fassen. Mit einem Winkel α zwischen 55° und 65° kann die Querbohrung soweit gegen distal verschoben werden, dass ihre Längsachse den Befestigungskopf in einer Distanz von weniger als 15 mm vor seinem Ende kreuzt. Eine weitere Querbohrung ist zur Verankerung des Radiusmarknagels in seiner Spitze vorgesehen. Als Werkstoff für den Radiusmarknagel sind körperverträgliche Metalle, zum Beispiel Chrom-Kobalt-Nickel-Legierungen nach ISO 5832-1 oder Titanlegierungen geeignet. Zum Einbringen des Marknagels wird zunächst mit einem Pfriem, dessen vorderer Bereich bezüglich Aussendurchmesser und Krümmung seiner Längsachse dem Radiusmarknagel entspricht, in der Nähe des Processus styloideus angesetzt, um den Marknagel in Form einer langgestreckten Krümmung zu eröffnen. Dies hat den Vorteil, dass die eingentlichen Gelenkflächen zu Scapahoid und zu Lunatum unberührt bleiben. Anschliessend kann der Radiusmarknagel an seinem Befestigungskopf mit einem Gleithammer in den Radiusknochen eingetrieben werden. Zur Befestigung des Gleithammers kann das gleiche Gewinde im Befestigungskopf wie für einen nachfolgend aufgesetzten Zielbügel verwendet werden. Zwischen Zielbügel und Befestigungskopf besteht zusätzlich eine formschlüssige Kopplung, die sicherstellt, dass Querbohrungen des Radiusmarknagels und zugehörige Zielbohrungen im Zielbügel miteinander fluchten. Zielvorichtung und Gleithammer können auch miteinander kombiniert sein.

Da die Querbohrungen im Radiusmarknagel nur einen Durchmeser von wenigen Millimetern haben können, ist es vorteilhaft, den Abstand zwischen Zielbügel und Querbohrung mit einer biegesteifen Bohrbüchse zu überbrücken. Gleichzeitig erhält ein Bohrer auf diese Weise eine wesentlich längere und genauere Führung, die mehr als das achtfache des Bohrerdurchmessers betragen kann. Die Bohrbüchsen sind abnehmbar, - damit sie die Befestigung des Zielbügels nicht hindern und nach dessen Befestigung soweit vorgetrieben werden können, dass sie mit ihrer geschärften Vorderkante eine Abstützung im Radiusknochen erhalten.

Im folgenden wird die Erfindung anhand von Ausführungsbeispielen beschrieben. Es zeigen:
- Fig. 1: Schematisch einen Längsschnitt durch einen in einem. Radiusknochen eingesetzten Marknagel;
- Fig. 2: Schematisch den Radiusmarknagel von Figur 1 in einer Seitenansicht auf die Ebene, in der die Krümmung seiner Längsachse liegt;
- Fig. 3: Schematisch eine vergrösserte, um 90° gedrehte Seitenansicht des Befestigungskopfes von Figur 2; und
- Fig. 4: Schematisch eine Seitenansicht eines Radiusmarknagels analog zu Figur 2 mit einer am Radiusmarknagel befestigten Zielvorrichtung.

Mit den Figuren ist ein Marknagel für den distalen Bereich eines Radiusknochens gezeigt. Der Marknagel besitzt einen Krümmungsradius R zwischen 100 und 1000 mm, damit er neben den distalen Gelenkflächen des Radiusknochens einbringbar ist, und eine schräggestellt Querbohrung, die den Marknagel in einer Distanz von weniger als 30 mm vor seinem Ende kreuzt, um auch Knochenstücke von Brüchen, die quer durch die distalen Lagerflächen verlaufen, zu fassen. Das Ende des Marknagels ist als Befestigungskopf für eine Zielvorrichtung für Querbohrungen ausgebildet.

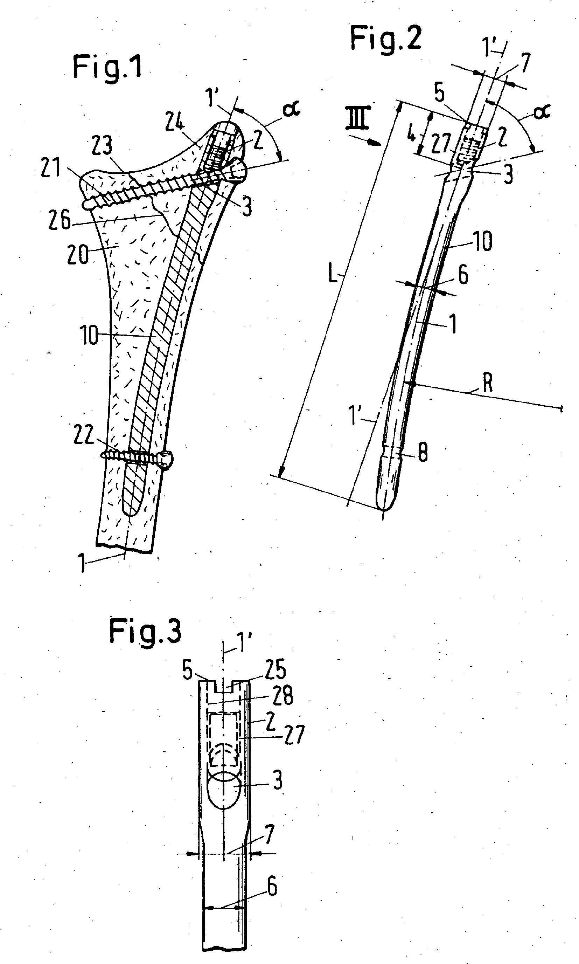

Die Hinweiszeichen sind in den Figuren gleich verwendet. In Figur 1 erstreckt sich ein Marknagel 10 in einen Radiusknochens 20 von dessen distalen Ende her und überbrückt eine Bruchstelle 26. Die Eintrittsöffnung für den Marknagel 20 ist ausserhalb der Gelenkpfannen 23, 24 für angrenzende Handgelenkknochen Lunatum und Scapahoid am Processus styloideus-gewählt. An der Spitze des Marknagels 10 ist eine Knochenschraube 22 quer eingebracht, um die Spitze im Radiusknochen 20 zu sichern. Der Marknagel 20 selbst ist in der Zeichenebene gekrümmt und an seinem gegen aussen gerichteten Ende mit einem Befestigungskopf 2 versehen, der eine gerade Längsachse 1' als Fortsetzung der Längsachse 1 des gekrümmten Bereiches aufweist. Eine zweite Querbohrung 3, welche in einem spitzen Winkel α die Längsachse 1' des Befestigungskopfes 2 kreuzt, ermöglicht es, den Marknagel auf der Eintrittsseite mit einer weiteren Knochenschraube 21 zu verankern und bei Brüchen, die durch die Lagerschalen 23, 24 gehen, die Bruchstücke zusätzlich zueinander zu fixieren.

Der Marknagel 10, wie er in Figur 2 und 3 dargestellt ist, besitzt eine ungestreckte Länge L von 100 mm und einen Krümmungsradius R von 350 mm. Der Durchmesser 6 des gekrümmten Bereiches beträgt 5 mm und der Durchmesser des Befestigungskopfes 7 beträgt 6 mm. Die schräggestellte Querbohrung 3 kreuzt den Befestigungskopf 2 in einem Abstand 4 von 12 mm von seinem äusseren Ende 5 in einem spitzen Winkel α von 59°. Die Querbohrung 3 selbst hat einen Durchmeser von 4 mm, während die Querbohrung 8 an der Spitze des Marknagels 10 einen Durchmesser von 2,7 mm besitzt. Das äussere Ende 5 ist als Auflagefläche ausgebildet, die durch eine Nut 25 unterbrochen ist, um eine Zielvorrichtung 9 bezüglich Drehung um die Längsachse 1' auszurichten. Auf der Innenseite ist eine zylindrische Bohrung 28 in Längsrichtung mit einem Gewinde 27 versehen, um die Zielvorrichtung 9 oder einen Gleithammer zu verankern. Im vorliegenden Beispiel wird ein Gleithammer, der einen vorstehenden Gewindezapfen aufweist, am Gewinde 27 des Befestigungskopfes angeschraubt und der Marknagel vorsichtig eingeschlagen.

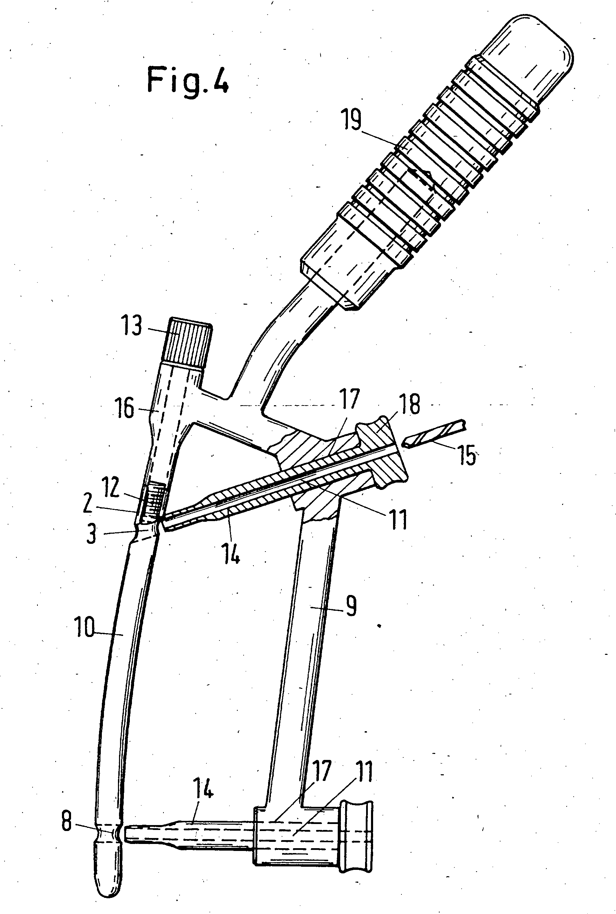

In Figur 4 ist ein Radiusmarknagel 10 mit seiner Zielvorrichtung 9 gekoppelt. Die Befestigung wird an einem Ansetzstück 16 mit einer Befestigungsschraube 12 vorgenommen, die mit ihrem Kopf 13 die Zielvorrichtung 9 hält und die Zielvorrichtung in einer vorgesehenen Position starr an den Marknagel koppelt. Die eigentliche Zielvorrichtung bildet einen U-förmigen Bügel 9 mit seitlich wegstehendem Griff 19. Fluchtend mit den Querbohrungen 3, 8 des Marknagels sind relativ grössere Führungsbohrungen 17 im Zielbügel 9 angebracht, in welche längsverschiebliche Bohrbüchsen 14 eingesetzt sind. Die Bohrbüchsen 14 verjüngen sich an ihrer Spitze zu einer dünnen, zugeschärften Hülse, die bis unmittelbar an den Marknagel 10 heranreicht und sich mit ihrer ringförmigen Schneide am Radiusknochen abstützen kann. Die relativ dünnen Bohrer 15, welche mit den Querbohrungen 3, 8 fluchtend auf einem Durchmeser von Zielbohrungen 11 die Knochenrinde vorbohren, sind bis unmittelbar an den Knochen geführt und können nicht aus der vorgesehenen Richtung verlaufen. Eine solche Führung ist bei einer zur Knochenwand schräg angeordneten Bohrung 3 besonders wichtig. Nach dem Vorbohren können die Bohrbüchsen 14 jeweils herausgezogen werden und durch die grösseren Führungsbohrungen 17 jeweils die passenden Knochenschrauben 22,21 angesetzt und eingedreht werden. Die Bohrbüchsen 14 besitzen jeweils einen Haltekopf 18, der die Zustellung in Längsrichtung begrenzt und ihr Herausziehen ermöglicht.

Grundsätzlich ist es auch möglich, die Zielvorrichtung mit einem Gleithammer zu kombinieren, indem beispielsweise der Kopf 13 der Befestigungsschraube 12 soweit nach oben verlängert ist, dass ein Innengewinde für den Gewindezapfen eines Gleithammers Platz hat. Wegen der relativ geringen Abmessungen des Marknagels muss dann darauf geachtet werden, dass die Zentrierflächen zwischen Ansetzstück 16 und Befestigungskopf 2 durch das Einschlagen nicht beschädigt werden.

## Patentansprüche

1. Radiusmarknagel mit einer Krümmung seiner Längsachse (1) entsprechend einem mittleren Krümmungsradius 100 mm < R < 1 000 mm, mit einem Befestigungskopf (2) für eine Zielvorrichtung (9) und mit einer schräggestellten, in der Ebene der Krümmung der Längsachse (1) des Radiusmarknagels (10) liegenden Querbohrung (3), die in einer Distanz (4) von weniger als 30 mm vom Ende (5) des Befestigungskopfes (2) entfernt die Längsachse (1) kreuzt.

2. Radiusmarknagel nach Anspruch 1, **dadurch gekennzeichnet, dass** er als Aussenabmessungen eine Länge L von 50 mm bis 200 mm und Durchmesser (6, 7) innerhalb eines Bereiches von 3 mm bis 10 mm aufweist.

3. Radiusmarknagel nach einem der Ansprüche 1 oder 2, **dadurch gekennzeichnet, dass** der Befestigungskopf (2) einen grösseren Durchmesser (7) als der restliche Nagel (10) aufweist.

4. Radiusmarknagel nach einem der Ansprüche 1 bis 3, **dadurch gekennzeichnet, dass** die schräggestellte Querbohrung (3) mit der Schaftachse (1') des Befestigungskopfes (2) einen Winkel α zwischen 40° und 70° bildet.

5. Radiusmarknagel nach Anspruch 4, **dadurch gekennzeichnet, dass** der Winkel α zwischen 55° und 65° liegt und dass die schräggestellte Querbohrung (3) die Längsachse (1) in einer Distanz (4) von weniger als 15 mm vom Ende (5) des Befestigungskopfes (4) kreuzt.

6. Radiusmarknagel nach einem der vorhergehenden Ansprüche, **dadurch gekennzeichnet, dass** er in der Nähe seiner Spitze eine weitere Querbohrung (8) aufweist.

7. Radiusmarknagel nach einem der vorhergehenden Ansprüche, **dadurch gekennzeichnet, dass** er aus einer Chrom-Kobalt-Nickel-Legierung beispielsweise aus einem Werkstoff nach ISO 5832-1 oder aus einer Titanlegierung hergestellt ist.

8. Zielvorrichtung mit einem Radiusmarknagel (10) nach einem der Ansprüche 1 bis 7, **dadurch gekennzeichnet, dass** sie in einer vorgegebenen Position am Befestigungskopf (2) des Radiusmarknagels (10) befestigbar ist und dass sie in dieser Position auf der Seite des Marknagels, auf welcher auch der Mittelpunkt für den Krümmungsradius R liegt, einen Bügel (9) mit Zielbohrungen (11) für die Querbohrungen (3, 8) am Radiusmarknagel (10) aufweist.

9. Zielvorrichtung mit einem Radiusmarknagel (10) nach Anspruch 8, **dadurch gekennzeichnet, dass** der Zielbügel (9) mit einer Befestigungsschraube (12) am Befestigungskopf (2) verankerbar ist, welche gleichzeitig einen zur Schlagübertragung ausgebildeten Schraubenkopf (13) aufweist.

10. Zielvorrichtung mit einem Radiusmarknagel (10) nach Anspruch 8 oder 9, **dadurch gekennzeichnet, dass** Führungsbohrungen (17) im Zielbügel (9) mit wesentlich grösseren Durchmessern als die Querbohrungen (3, 8) des Radiusmarknagels (10) angebracht sind, und dass Bohrbüchsen (14) in die Führungsbohrungen (17) einsetzbar sind, welche einen Bohrer (15) über eine Länge von mehr als dem achtfachen des Bohrerdurchmessers führen.

## Claims

1. An intramedullary nail for the radius having a curvature of its longitudinal axis (1) corresponding to a mean curvature radius 100 mm < R < 1,000 mm, having a fastening head (2) for a target apparatus (9) and having an inclined transverse bore (3) which lies in the plane of the curvature of the longitudinal axis (1) of the intramedullary nail for the radius (10) and which intersects the longitudinal axis (1) at a distance (4) of less than 30 mm from the end (5) of the fastening head (2).

2. An intramedullary nail for the radius in accordance with claim 1, **characterized in that** it has as outer dimensions a length L of 50 mm to 200 mm and a diameter (6, 7) with a range of 3 mm to 10 mm.

3. An intramedullary nail for the radius in accordance with one of claims 1 or 2, **characterized in that** the fastening head (2) has a larger diameter (7) than the remaining nail (10).

4. An intramedullary nail for the radius in accordance with any one of the claims 1 to 3, **characterized in that** the inclined transverse bore (3) forms an angle α between 40° and 70° with the shaft axis (1') of the fastening head (2).

5. An intramedullary nail for the radius in accordance with claim 4, **characterized in that** angle α is between 55° and 65°; and **in that** the inclined transverse bore (3) intersects the longitudinal axis (1) at a distance (4) of less than 15 mm from the end (5) of the fastening head (2).

6. An intramedullary nail for the radius in accordance with any one of the preceding claims, **characterized in that** it has a further transverse bore (8) in the proximity of its tip.

7. An intramedullary nail for the radius in accordance with any one of the preceding claims, **characterized in that** it is manufactured from a chromium-cobalt-nickel alloy, for example from a material in accordance with ISO 5832-1, or from a titanium alloy.

8. A target apparatus having an intramedullary nail for the radius (10) in accordance with any one of the claims 1 to 7, **characterized in that** it is fastenable to the fastening head (2) of the intramedullary nail for the radius (10) in a predefined position; and **in that** it has a hoop (9) having target bores (11) for the transverse bores (3, 8) at the intramedullary nail for the radius (10) in this position at the side of the intramedullary nail on which the center for the radius of curvature R lies.

9. A target apparatus having an intramedullary nail for the radius (10) in accordance with claim 8, **characterized in that** the target hoop (9) can be anchored to the fastening head (2) by a fastening screw (12) which simultaneously has a screw head (3) formed for a transfer of blows.

10. A target apparatus having an intramedullary nail for the radius (10) in accordance with claim 8 or claim 9, **characterized in that** guide bores (17) having substantially larger diameters than the transverse bores (3, 8) of the intramedullary nail for the radius (10) are applied in the target hoop (9); and **in that** bore bushes (14) can be inserted into the guide bores (17) which guide a drill (15) over a length of more than eight times the drill diameter.

## Revendications

1. Clou médullaire pour le radius avec une courbure de son axe longitudinal (1) correspondant à un rayon de courbure moyen tel que 100 mm < R < 1000 mm, avec une tête de fixation (2) pour un dispositif de visée (9) et avec un perçage transversal (3) disposé en oblique et situé dans le plan de la courbure de l'axe longitudinal (1) du clou médullaire pour le radius (10), perçage transversal qui croise l'axe longitudinal (1) à une distance (4) éloignée de moins de 30 mm depuis l'extrémité (5) de la tête de fixation (2).

2. Clou médullaire pour le radius selon la revendication 1, **caractérisé en ce qu'**il présente comme dimensions extérieures une longueur L de 50 mm à 200 mm et un diamètre (6, 7) à l'intérieur d'une plage de 3 mm à 10 mm.

3. Clou médullaire pour le radius selon l'une des revendications 1 ou 2, **caractérisé en ce que** la tête de fixation (2) présente un diamètre (7) plus grand que le reste du clou (10).

4. Clou médullaire pour le radius selon l'une des revendications 1 à 3, **caractérisé en ce que** le perçage transversal (3) disposé en oblique forme avec l'axe de la tige (1') de la tête de fixation (2) un angle α entre 40° et 70°.

5. Clou médullaire pour le radius selon la revendication 4, **caractérisé en ce que** l'angle α est compris entre 55° et 65°, et **en ce que** le perçage transversal (3) disposé en oblique croise l'axe longitudinal (1) à une distance (4) de moins de 15 mm de l'extrémité (5) de la tête de fixation (4).

6. Clou médullaire pour le radius selon l'une des revendications précédentes, **caractérisé en ce qu'**il comporte un autre perçage transversal (8) au voisinage de sa pointe.

7. Clou médullaire pour le radius selon l'une des revendications précédentes, **caractérisé en ce qu'**il est fabriqué en un alliage chrome-cobalt-nickel, par exemple en un matériau selon la norme ISO 5832-1, ou en un alliage de titane.

8. Dispositif de visée comprenant un clou médullaire pour le radius (10) selon l'une des revendications 1 à 7, **caractérisé en ce qu'**il est susceptible d'être fixé sur la tête de fixation (2) du clou médullaire pour le radius (10) dans une position prédéterminée et **en ce que** dans cette position, il comprend, sur le côté du clou médullaire sur lequel se trouve également le centre du rayon de courbure R, un arceau (9) avec des perçages de visée (11) pour les perçages transversaux (3, 8) sur le clou médullaire pour le radius.

9. Dispositif de visée comprenant un clou médullaire pour le radius (10) selon la revendication 8, **caractérisé en ce que** l'arceau de visée (9) est susceptible d'être ancré sur la tête de fixation (2) avec une vis de fixation (12) qui comporte simultanément une tête de vis (13) réalisée pour la transmission de coups.

10. Dispositif de visée comprenant un clou médullaire pour le radius (10) selon la revendication 8 ou 9, **caractérisé en ce que** les perçages de guidage (17) sont ménagés dans l'arceau de visée (9) avec des diamètres sensiblement plus grands que les perçages transversaux (3, 8) du clou médullaire pour le radius (10), et **en ce que** des douilles de perçage (14) sont susceptibles d'être mises en place dans les perçages de guidage (17), lesdites douilles guidant un foret (15) sur une longueur de plus de huit fois le diamètre du foret.
